# EUROPEAN PATENT SPECIFICATION

(11) **EP 3 393 558 B1**
(45) Date of publication and mention of the grant of the patent: **28.10.2020**
(21) Application number: 16815570.3
(22) Date of filing: 21.12.2016
(51) Int. Cl.: A61M 11/06, A61M 16/04, A61M 16/08

(54) **NASOPHARYNGEAL CANNULA**
NASEN-RACHENKANÜLE
CANULE NASOPHARYNGÉE

(30) Priority: 23.12.2015 IT UB20159256
(43) Date of publication of application: 31.10.2018
(73) Proprietor: Deas S.R.L., 48014 Castel Bolognese (IT)
(72) Inventor: SCARDOVI, Domenico, 48014 Castel Bolognese (IT); SORBELLO, Massimiliano, 95127 Catania (IT)
(74) Representative: Modiano, Micaela Nadia
(86) International application number: PCT/EP2016/082162
(87) International publication number: WO 2017/108940

(56) References cited:
- EP-A1- 2 204 206
- WO-A1-2012/103490
- WO-A1-2013/192121
- WO-A2-2014/163110
- GB-A- 2 391 812
- US-A1- 2010 069 770

## Description

The present invention relates to a nasopharyngeal cannula.

A nasopharyngeal cannula is constituted by a soft tubular element that can be inserted through the nose up to the posterior pharynx.

These cannulas have the purpose of performing a forced or assisted ventilation to a patient, facilitating his/her physiological process known as respiration.

Respiration is a process by means of which oxygen and carbon dioxide are exchanged between the animal tissues and the external environment. It is composed of ventilation, with which the air that is present in the alveoli is refreshed, the diffusion of oxygen from the alveoli to capillary blood, and the release of carbon dioxide. The respiratory system is constituted by a system of conduction pathways, composed of trachea, bronchi and non-respiratory bronchioles, at the level of which respiratory exchanges do not occur, and the respiratory terminal unit, composed of respiratory bronchioles, alveolar ducts and alveoli, where instead gaseous exchanges occur.

A patient subjected to artificial and/or assisted ventilation must be monitored in order to check that his/her body receives a suitable quantity of oxygen. One of the most effective and precise methods for verifying that the patient is oxygenated correctly provides for measuring the content of carbon dioxide in the exhaled gas (in particular the so-called "end-exhaled carbon dioxide" value). From this parameter it is possible to estimate the correct respiratory functionality, checking whether the parameters set for ventilation are suitable or whether it is necessary to change them (for example, an increase/decrease of the percentage of oxygen in the dispensed mixture).

Nasopharyngeal cannulas of the known type provide for the presence of a duct, provided along the tubular walls of the cannula and open at an internal central cross-section thereof, through which it is possible to aspirate the gas in order to measure the carbon dioxide content.

With the purpose of holding carbon dioxide monitoring systems near the patient's breathing orifices, US2010069770 discloses an end-tidal CO₂ monitoring tube holder to be used with nasal cannulas.

However, this type of measurement is often imprecise, since the region where the exhaled gas is drawn is highly subjected to contamination on the part of the mixture dispensed by the cannula to the patient and therefore the measurement is rendered imprecise by phenomena of dilution, "washing" and mixing with the (oxygen-rich) mixture provided by the ventilation apparatus.

Furthermore, it should be specified that the insertion of a nasopharyngeal cannula is unpleasant and in some cases can even be painful.

Secondarily, it should be clarified that the cannula, even after it has been positioned correctly, can induce sneezing or coughing in the patient: these phenomena are particularly problematic if the intubated patient is simultaneously subjected to other diagnostic investigations or therapeutic activities.

For example, nasopharyngeal cannulas are used during gastroscopies: it is evident that if the patient sneezes or coughs during a gastroscopy this can cause problems and/or force the gastroscopic instruments against the mucosae (with consequent possible abrasions or painful stresses for the patient).

The aim of the present invention is to solve the problems described above, by proposing a nasopharyngeal cannula that allows precise measurement of the content of carbon dioxide that is present in the gas exhaled by the patient who is wearing it, and in particular measurement of the so-called end-exhaled carbon dioxide value.

Within this aim, an object of the invention is to propose a nasopharyngeal cannula that allows to minimize discomfort for the patient.

Another object of the invention is to propose a nasopharyngeal cannula that minimizes the risk of sneezing or coughing.

Another object of the invention is to propose a nasopharyngeal cannula with geometries and with a structure that are substantially different from those of the known type.

A further object of the present invention is to provide a nasopharyngeal cannula that has modest costs, is relatively simple to provide in practice and is safe in application.

This aim and these and other objects which will become better apparent hereinafter are achieved by a nasopharyngeal cannula having the features of attached claim 1.

This aim and these objects are also achieved by means of a nasopharyngeal ventilation kit having the features of attached claim 11.

Further aspects are disclosed by the attached dependent claims 2 to 10.

Further characteristics and advantages of the invention will become better apparent from the description of a preferred but not exclusive embodiment of the nasopharyngeal cannula according to the invention, illustrated by way of nonlimiting example in the accompanying drawings, wherein:

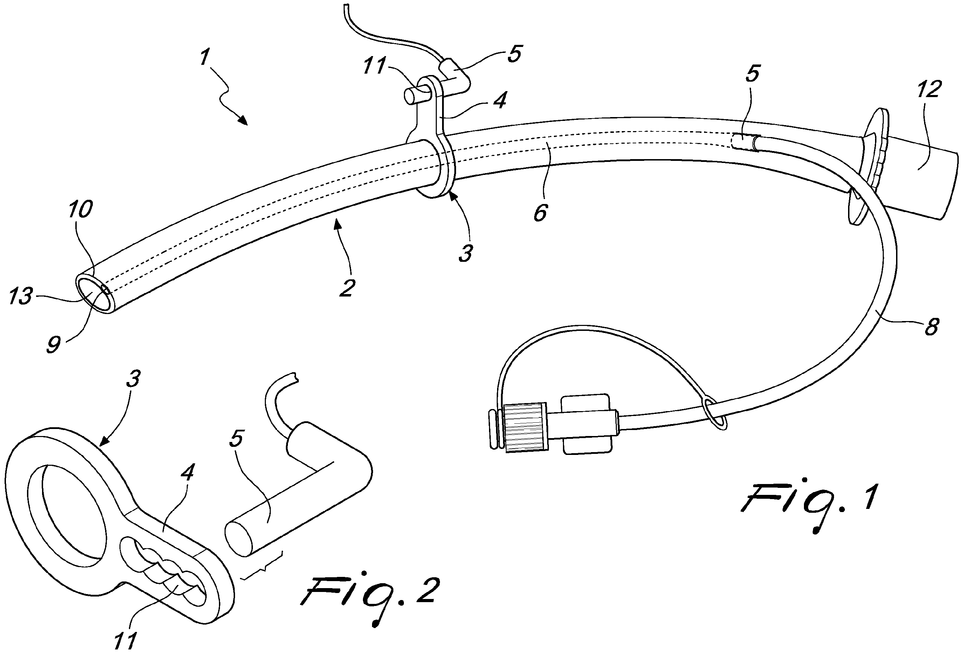

Figure 1 is a perspective view of a nasopharyngeal cannula according to the invention;
Figure 2 is a perspective view of a bracket of a nasopharyngeal cannula according to the invention;

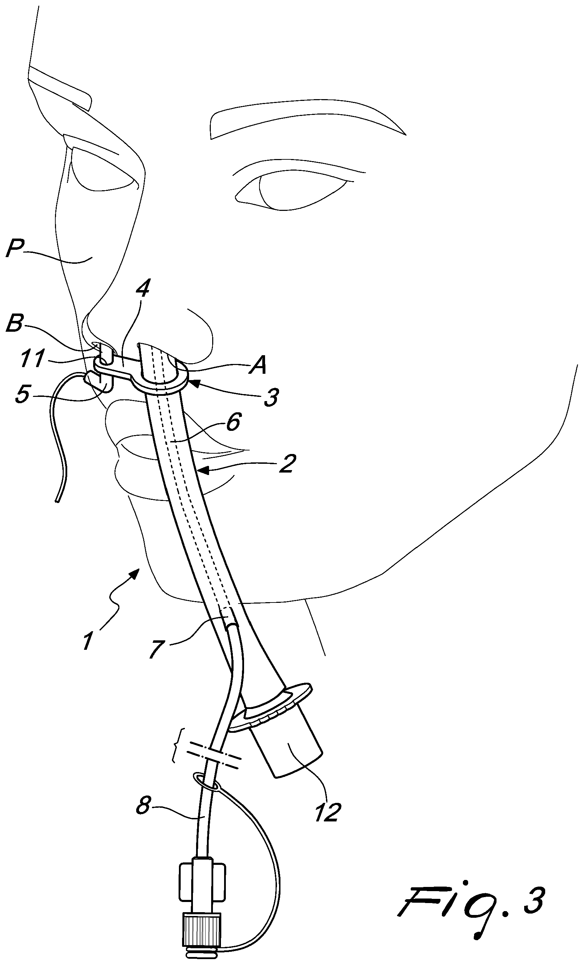

Figure 3 is a schematic front view of a patient subjected to ventilation by means of a nasopharyngeal cannula according to the invention;
Figure 4 is a front view of a possible embodiment of a bracket of a nasopharyngeal cannula according to the invention;
Figure 5 is a front view of a further embodiment of a bracket of a nasopharyngeal cannula according to the invention;
Figure 6 is a front view of a further embodiment of a bracket of a nasopharyngeal cannula according to the invention;

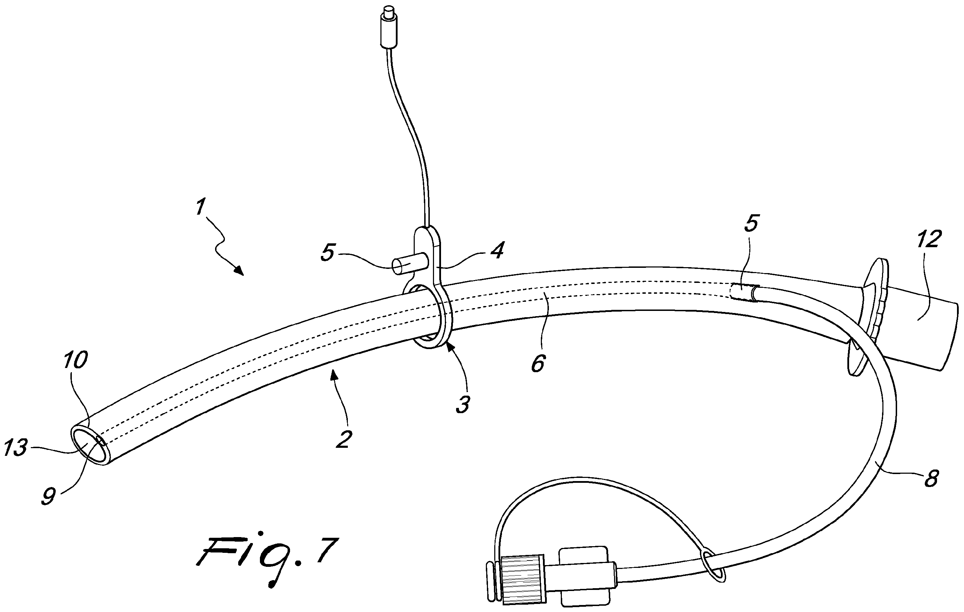

Figure 7 is a perspective view of a further embodiment of a nasopharyngeal cannula according to the invention in which the sensor is integral with the bracket.

With reference to the figures, the reference numeral 1 generally designates a nasopharyngeal cannula.

The nasopharyngeal cannula 1 comprises a main tubular body 2 made of soft and deformable material: the deformability and softness of the tubular body 2 are necessary in order to prevent it being able to damage the mucosae of the patient and to ensure that it causes the least possible discomfort to said patient.

The tubular body 2 comprises a bracket 3, which is provided with at least one support 4 for a sensor 5.

The bracket 3 can be associated with the side walls of the tubular body 2.

The possibility is provided from the outset to provide a bracket 3 which is fixed (which constitutes a collar that is integral with the external surface of the tubular body 2), or can slide and can be oriented with respect to the tubular body 2 (the bracket 3 can optionally also be loose with respect to the tubular body 2 and in this case its locking will be ensured by fixing to the nostril A of a patient P and/or by the presence of additional specific components): the second case is more versatile and allows a simpler insertion thereof in the nostril A of a patient P.

The possibility to provide a bracket 3 that is formed monolithically or coupled non-detachably to the sensor 5 is not excluded.

The tubular body 2 further comprises at least one duct 6, the initial end 7 of which can be associated, by means of at least one respective connecting tube 8, to external operating units and the terminal end 9 of which is constituted by an opening on the front 10 of the terminal edge of the tubular body 2.

The duct 6 will be constituted by a true longitudinal channel which is internal to the side walls of the tubular body 2, although the provision of a version in which the duct 6 is a small tube that is integral with the internal surface of the tubular body 2 is not excluded.

In the configuration for use, the tubular body 2 is inserted in a nostril A of a patient P until its front 10 aligns with the pharynx of the patient P.

The sensor 5, assigned to measuring the percentage of carbon dioxide that is present in the exhaled gas, during use is at least partially inserted in the other nostril B of the patient P and therefore is struck by the exhaled flow.

In particular, the sensor 5 must be capable of monitoring continuously the parameter known as EtCO₂ (end-exhaled carbon dioxide), i.e., checking:
- the conditions of the patients, especially those placed in positions in which it can be difficult to check their status with other systems;
   - any hypoventilation caused by sedation and/or analgesia;
   - the conditions that will indicate the appropriate dosage of drugs during invasive procedures.

The sensor 5 is therefore preferably a capnometric sensor, i.e., suitable to measure the carbon dioxide in the air exhaled by the patient P. The sensor 5 can be connected to a capnograph in order to provide a capnogram.

The sensor 5 can be constituted by a cannula through which it is possible to draw the gas exhaled by the patient P and send it to a true capnometric transducer.

The fact that the sensor 5 faces (and is partially inserted in) the nostril B ensures that the measurement performed thereby is not compromised by dilution problems caused by the mixing of the exhaled gases with the mixture introduced through the tubular body 2 (as instead occurs in nasopharyngeal cannulas of the known type).

It is deemed useful to note that according to an embodiment of unquestionable practical interest, the bracket 3 can be coupled validly so that it can slide and rotate to the tubular body 2 (as mentioned earlier): this allows its arrangement in any geometric configuration with respect to the tubular body 2. This characteristic is particularly useful during insertion, since the medical staff can choose the nostril A or B into which the tubular body 2 is to be inserted and arrange accordingly the bracket 3 on the opposite side, at the desired height, in order to allow the easy at least partial insertion of the sensor 5 in the other nostril B or A.

Furthermore, it is specified that the support 4 is provided in such a manner as to protrude from the bracket 3 in a substantially radial direction with respect to the tubular body 2.

The support 4 preferably comprises a seat 11 for the accommodation of the sensor 5.

The seat 11 can have a shape that is complementary to the shape of the sensor 5 in order to accommodate it stably.

It is not excluded that the support might comprise a plurality of seats 11 within which the sensor 5 is accommodated: in this case, the medical staff chooses the seat 11 within which the sensor 5 is to be inserted in order to achieve the best alignment thereof with the nostril B of the patient into whom it must be at least partially inserted.

According to an embodiment that is particularly versatile and simple to use, the seat 11 can conveniently have a slotted shape for the stable accommodation of the sensor 5 according to a plurality of different configurations of different distance of the sensor 5 from the outer surface of the tubular body 2.

This specific version can be even more convenient if the internal surface of the slotted seat 11 is contoured or toothed in order to ensure a series of contiguous configurations for stable accommodation of the sensor 5.

According to a particularly efficient embodiment, the bracket 3 is substantially C-shaped and is made of elastically deformable material, for detachable coupling, as a consequence of its elastic deformation, to the tubular body 2.

In practice it is possible to couple the bracket 3 on the tubular body 2 simply by forcing it against its surface, with consequent elastic deformation thereof (the wings of the C-shape will be widened, allowing the passage of the tubular body 2). The advantage of this embodiment resides in the possibility to associates the bracket 3 with the tubular body 2 only following its insertion in the nostril A of the patient P, thus allowing medical staff to perform insertion in optimum conditions (without hindrances).

Furthermore, the possibility is not excluded to ensure rapid replacement of the bracket 3 with another one if needed.

In its simplest embodiment, the bracket 3 is constituted by a plate having any shape, provided with a central hole the shape and dimensions of which are complementary to those of the tubular body 2 for the sliding accommodation of the tubular body 2. The support 4 extends radially (laterally) from said bracket.

Furthermore, it is deemed useful to specify that the at least one tube 8 for connection of the at least one duct 6 validly comprises a terminal for coupling to a respective unit for dispensing substances such as drugs, anesthetics, analgesics, water and mixtures thereof.

The possibility to dispense substances of the listed type is particularly useful in relation to the possibility to minimize discomfort for the patient, with consequent reduction of physiological reactions such as coughing and/or sneezing, which might complicate other diagnostic and/or therapeutic activities in progress on the patient.

It is specified that the terminal opening 9 of the at least one respective duct 6 can conveniently comprise a nozzle for atomizing the substances dispensed thereby.

This characteristic allows to reduce the quantity of dispensed substances (be they pharmaceutical, medical, anesthetic, analgesic and the like) since their atomization allows optimum distribution on mucosae and tissues.

According to a particular embodiment that is particularly interesting from the constructive standpoint, the atomization nozzle of the second opening 9 is a hollow body that is inserted within the at least one duct 6 and is arranged substantially at the respective terminal opening 9.

In this case, the internal cavity of said hollow body has a shape that first converges and then diverges, of the type of a Venturi tube, for the acceleration of the substances that flow inside it with consequent better atomization thereof.

The hollow body can be fixed inside the duct 6, at the opening 9, as a consequence of an elastic deformation of the duct 6 (elastic extension) or can be coupled stably by means of adhesive and/or bonding substances.

It should be specified that the main body 2 is preferably provided by means of a thermoplastic polymer and comprises a nontraumatic, i.e., soft and rounded, distal tip.

These characteristics allow to minimize the discomfort of the patient who has to be intubated with the cannula 1.

The tubular body 2 comprises, in its proximal portion, at least one connector 12 for connection to a forced/assisted ventilation element.

The shape and dimensions of the connector 12 are complementary to those of the coupling terminal of the forced/assisted ventilation element.

Furthermore, it is specified that the length and outside diameter of the tubular body 2 are variable as a function of the anatomical parameters of the patient to be intubated, the possibility being provided to provide a family of nasopharyngeal cannulas 1 having progressively larger sizes starting from a minimum size (for example suitable to be used on neonatal patients) up to a maximum size (for very tall patients and/or patients having particular anatomical configurations).

Furthermore, it should be specified that the lumen 13 inside the tubular body 2 has a diameter that is suitable for the passage of endoscopic instruments.

The internal lumen 13 is in fact generally used only to convey gaseous ventilation mixtures; however, one cannot exclude that in some cases it might be necessary to insert endoscopic instruments for diagnostic and/or therapeutic purposes.

When endoscopic instruments are inserted in the internal lumen 13 of the tubular body 2 it is possible to provide for the at least one duct 6 to be used to dispense oxygen in order to ensure that the patient is in any case ensured a sufficient ventilation.

In this use case it is particularly useful to have a number of ducts 6 greater than one (at least two ducts 6) in order to be able to provide a sufficient quantity of oxygen to the patient.

Likewise, the tubular body 2 can comprise a third duct also for accommodating a light source, such as an LED with respective power supply circuit, at least one optical fiber for conveying a beam of light produced by an external source, and the like.

In this case, the light source can provide support for any endoscopic activities performed by inserting the instruments in the lumen 13.

In order to render the arrangement of the cannula 1 in the nasopharyngeal cavity of the patient more precise, the tubular body 2 comprises, on its outer surface, a graduated scale in order to estimate the length of its portion that is inserted within the nose.

The present invention also relates to a nasopharyngeal ventilation kit, which comprises:
- a main tubular body 2 made of soft and deformable material, provided with a nontraumatic, i.e., soft and rounded, distal tip.

The tubular body comprises at least one duct 6, the initial end 7 of which can be associated, by means of at least one respective connecting tube 8, with external operating unit and the terminal end 9 of which is constituted by an opening on the front 10 of the terminal edge of the tubular body 2;
- a bracket 3, provided with at least one support 4 for a sensor 5 that is assigned to measuring the percentage of carbon dioxide that is present in the exhaled gas.

The kit, in the configuration for use, is installed so that the bracket 3 is associated with the side walls of the tubular body 2 inserted within a nostril A of a patient P; the sensor 5 is at least partially inserted in the other nostril B of the patient P and is struck by the exhaled flow, allowing precise measurement of the intake carbon dioxide (a parameter by means of which the gaseous ventilation mixture is adjusted in order to optimize the respiration of the patient P).

The present invention extends its protective scope also to a bracket 3 for nasopharyngeal ventilation cannulas that is suitable for association with a main tubular body 2 made of soft and deformable material, provided with a nontraumatic, i.e., soft and rounded, distal tip.

The bracket 3 can validly comprise at least one support 4 for a sensor 5 that is assigned to measuring the percentage of carbon dioxide that is present in the exhaled gas.

The support 4 comprises a seat 11 for accommodating the sensor 5: in practice, the sensor 5 can be locked within the seat 11, providing a coupling by interference, friction or of the elastic type.

In the configuration for use, the bracket 3 can be associated advantageously with the side walls of the tubular body 2 (which constitutes the actual nasopharyngeal cannula) inserted within a nostril A of a patient P.

In this case, the sensor 5 is at least partially inserted in the other nostril B of the patient P and is struck by the exhaled flow.

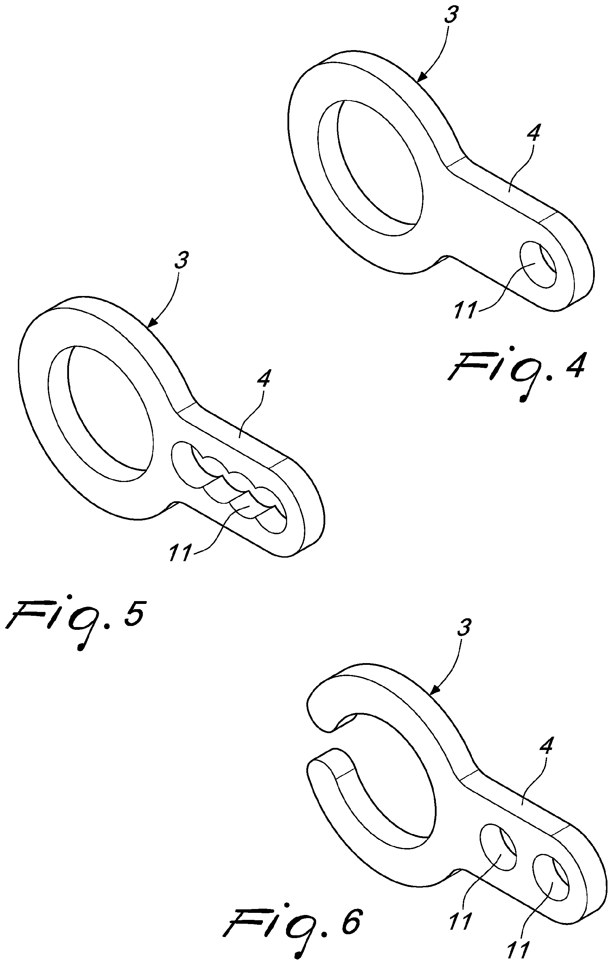

The support 4 protrudes from the bracket 3 in a substantially radial direction with respect to the tubular body 2 to which it is coupled, so that the seat 11 for the sensor 5 faces and is proximate to the free nostril B (with respect to the one engaged by the tubular body 2). Some possible embodiments of the bracket 3 are shown by way of nonlimiting example in Figures 4, 5 and 6.

Advantageously, the present invention solves the problems described earlier, proposing a nasopharyngeal cannula 1 that allows a precise measurement of the content of carbon dioxide that is present in the gas exhaled by the patient who is wearing it, and in particular the measurement of the so-called end-exhaled carbon dioxide value. This measurement is particularly precise, since the sensor 5 is at least partially inserted within a nostril B of the patient P and therefore is struck directly by the stream of gas exhaled thereby, allowing a precise estimate of the percentage of carbon dioxide contained therein.

Advantageously, the nasopharyngeal cannula 1 allows to minimize discomfort for the patient: it in fact provides for at least one duct 6 which is provided with a respective opening 9 for atomizing pharmaceutical, anesthetic, analgesic and similar substances. The administration by atomization of these substances reduces discomfort for the patient.

Positively, the nasopharyngeal cannula 1 minimizes the risk of sneezing or coughing.

As already mentioned earlier, in fact, the administration by atomization of the listed substances make it unlikely for the cannula 1 to induce coughing and/or sneezing in the patient.

Validly, the nasopharyngeal cannula 1 has geometries and a structure that are substantially different from those of the known type, defining specific standards that are suitable to make them immediately identifiable for medical staff.

Conveniently, the nasopharyngeal cannula 1 is relatively simple to provide in practice and can be manufactured industrially with substantially modest costs; this makes it a product of assured application.

The invention thus conceived is susceptible of numerous modifications and variations, all of which are within the scope of the appended claims; all the details may further be replaced with other technically equivalent elements.

In the exemplary embodiments shown, individual characteristics, given in relation to specific examples, may actually be interchanged with other different characteristics that exist in other exemplary embodiments.

In practice, the materials used, as well as the dimensions, may be any according to the requirements and the state of the art.

Where technical features mentioned in any claim are followed by reference signs, those reference signs have been included for the sole purpose of increasing the intelligibility of the claims and accordingly such reference signs do not have any limiting effect on the interpretation of each element identified by way of example by such reference signs.

## Claims

1. A nasopharyngeal cannula comprising a main tubular body (2) made of soft and deformable material, a sensor (5), and a bracket (3), said bracket (3) being provided with at least one support (4) for said sensor (5), in the configuration for use said tubular body (2) being inserted within a nostril (A) of a patient (P) until a front (10) is aligned with the pharynx of said patient (P), and said sensor (5), preset to measure the percentage of carbon dioxide that is present in the exhaled gas, being inserted at least partially in the other nostril (B) of the patient (P) and being struck by the exhaled flow, said bracket (3) being coupled slidingly and rotatably to the lateral walls of said tubular body (2) for its arrangement in any geometric configuration with respect to said tubular body (2), said support (4) protruding from said bracket (3) in a substantially radial direction with respect to said tubular body (2), said support (4) comprising a seat (11) for the accommodation of said sensor (5), said sensor (5) being constituted by a cannula through which it is possible to draw the gas exhaled by the patient (P) and send it to a true capnometric transducer.

2. The nasopharyngeal cannula according to claim 1, **characterized in that** said tubular body (2) is provided with at least one duct (6) that has the initial end (7) associable, by means of at least one respective connecting tube (8), with external operating units and the terminal end (9) constituted by an opening on the front (10) of the terminal edge of said tubular body (2).

3. The nasopharyngeal cannula according to claim 1, **characterized in that** said seat (11) has a slotted shape for the stable accommodation of said sensor (5) according to a plurality of different configurations of different distance of said sensor (5) from the external surface of said tubular body (2).

4. The nasopharyngeal cannula according to claim 1, **characterized in that** said bracket (3) is substantially C-shaped and is made of elastically deformable material, for detachable coupling, as a consequence of an elastic deformation thereof, to said tubular body (2).

5. The nasopharyngeal cannula according to claim 1, **characterized in that** said bracket (3) is constituted by a plate of any shape provided with a central hole with a shape and dimensions that are complementary to those of said tubular body (2) for the sliding accommodation of said tubular body (2).

6. The nasopharyngeal cannula according to claim 2, **characterized in that** said at least one connecting tube (8) of the at least one duct (6) comprises a terminal for coupling to a respective unit for dispensing substances such as drugs, anesthetics, painkillers, water and mixtures thereof.

7. The nasopharyngeal cannula according to claim 2, **characterized in that** the terminal opening (9) of the at least one duct (6) comprises a nozzle for atomizing the substances delivered thereby.

8. The nasopharyngeal cannula according to claim 7, **characterized in that** said atomization nozzle is a hollow body that is inserted within the at least one duct (6) and is arranged substantially at the respective terminal opening (9), the internal cavity of said hollow body has a shape that initially converges and then diverges, such as a Venturi tube, for the acceleration of the substances that flow inside it with consequent better atomization thereof.

9. The nasopharyngeal cannula according to claim 1, **characterized in that** said main body (2) is made of thermoplastic polymers and comprises a nontraumatic, i.e., soft and rounded, distal tip.

10. The nasopharyngeal cannula according to claim 7, **characterized in that** it comprises a third duct for accommodating a light source, such as an LED with a respective power supply circuit, at least one optical fiber for conveying a beam of light produced by an external source, and the like.

11. A nasopharyngeal ventilation kit, comprising
- a main tubular body (2) made of soft and deformable material, provided with a nontraumatic, i.e., soft and rounded, distal tip;
- a sensor (5) preset to measure the percentage of carbon dioxide that is present in the exhaled gas;
- a bracket (3), provided with at least one support (4) for a said sensor (5); and
- a true capnometric transducer;
- in use, said bracket (3) being coupled slidingly and rotatably to the lateral walls of said tubular body (2) for its arrangement in any geometric configuration with respect to said tubular body (2);
- said support (4) protruding from said bracket (3) in a substantially radial direction with respect to said tubular body (2), said support (4) comprising a seat (11) for the accommodation of said sensor (5);
in the configuration for use with said tubular body (2) inserted in a nostril (A) of a patient (P) until a front (10) is aligned with the pharynx of said patient (P), said sensor (5) is inserted at least partially in the other nostril (B) of the patient (P) and being struck by the exhaled flow, said sensor (5) being constituted by a cannula through which it is possible to draw the gas exhaled by the patient (P) and send it to said true capnometric transducer.

## Patentansprüche

1. Eine Nasen-Rachenkanüle, die einen schlauchförmigen Hauptkörper (2) aus weichem und verformbarem Material, einen Sensor (5) und eine Klammer (3) umfasst, wobei die Klammer (3) mit mindestens einem Träger (4) für den Sensor (5) ausgestattet ist, wobei der schlauchförmige Körper (2) in der Nutzungskonfiguration in ein Nasenloch (A) eines Patienten (P) eingeführt wird, bis ein vorderes Ende (10) mit dem Rachen des Patienten (P) fluchtet; und wobei der Sensor (5), der voreingestellt ist, um den Prozentsatz an Kohlendioxid zu messen, der in dem ausgeatmeten Gas vorhanden ist, zumindest teilweise in das andere Nasenloch (B) des Patienten (P) eingeführt und von dem ausgeatmeten Luftstrom getroffen wird; wobei die Klammer (3) verschiebbar und drehbar mit den Seitenwänden des schlauchförmigen Körpers (2) gekoppelt ist zum Zwecke ihrer Anordnung in einer beliebigen geometrischen Konfiguration im Verhältnis zu dem schlauchförmigen Körper (2); wobei der Träger (4) von der Klammer (3) in einer im Wesentlichen radialen Richtung im Verhältnis zu dem schlauchförmigen Körper (2) absteht; wobei der Träger (4) einen Sitz (11) für die Aufnahme des Sensors (5) umfasst, wobei der Sensor (5) aus einer Kanüle besteht, durch welche das vom Patienten (P) ausgeatmete Gas entnommen werden und an einen echten kapnometrischen Wandler geleitet werden kann.

2. Die Nasen-Rachenkanüle gemäß Anspruch 1, **dadurch gekennzeichnet, dass** der schlauchförmige Körper (2) mit mindestens einer Leitung (6) versehen ist, deren Anfangsende (7) über mindestens einen entsprechenden Verbindungsschlauch (8) mit externen Arbeitseinheiten verbunden werden kann und deren terminales Ende (9) in einer Öffnung am vorderen Ende (10) des terminalen Randes des schlauchförmigen Körpers (2) besteht.

3. Die Nasen-Rachenkanüle gemäß Anspruch 1, **dadurch gekennzeichnet, dass** der Sitz (11) eine Schlitzform hat, für die feste Aufnahme des Sensors (5) in einer Vielzahl verschiedener Konfigurationen verschiedenen Abstands des Sensors (5) von der äußeren Oberfläche des schlauchförmigen Körpers (2).

4. Die Nasen-Rachenkanüle gemäß Anspruch 1, **dadurch gekennzeichnet, dass** die Klammer (3) im Wesentlichen C-förmig ist und aus elastisch verformbarem Material besteht, zur lösbaren Kopplung, infolge einer elastischen Verformung derselben, mit dem schlauchförmigen Körper (2).

5. Die Nasen-Rachenkanüle gemäß Anspruch 1, **dadurch gekennzeichnet, dass** die Klammer (3) aus einer Platte mit beliebiger Form besteht, versehen mit einem zentralen Loch mit Form und Abmessungen, die zur gleitenden Aufnahme des schlauchförmigen Körpers (2) komplementär zu denjenigen des schlauchförmigen Körpers (2) sind.

6. Die Nasen-Rachenkanüle gemäß Anspruch 2, **dadurch gekennzeichnet, dass** der mindestens eine Verbindungsschlauch (8) der mindestens einen Leitung (6) ein Endstück zur Kopplung mit einer entsprechenden Einheit für die Abgabe von Substanzen wie Arzneimitteln, Anästhetika, Schmerzmitteln, Wasser und Mischungen davon umfasst.

7. Die Nasen-Rachenkanüle gemäß Anspruch 2, **dadurch gekennzeichnet, dass** das terminale Ende (9) der mindestens einen Leitung (6) eine Düse zum Zerstäuben der davon abgegebenen Substanzen umfasst.

8. Die Nasen-Rachenkanüle gemäß Anspruch 7, **dadurch gekennzeichnet, dass** die Zerstäubungsdüse ein Hohlkörper ist, der in die mindestens eine Leitung (6) eingesetzt und im Wesentlichen am entsprechenden terminalen Ende (9) angeordnet ist; der innere Hohlraum des Hohlkörpers hat eine Form, die zunächst zusammenläuft und sich dann verzweigt, wie zum Beispiel eine Venturi-Düse, zur Beschleunigung der Substanzen, die in ihm strömen, mit daraus folgender besserer Zerstäubung derselben.

9. Die Nasen-Rachenkanüle gemäß Anspruch 1, **dadurch gekennzeichnet, dass** der Hauptkörper (2) aus thermoplastischen Polymeren besteht und eine nichttraumatische, d. h. weiche und gerundete, distale Spitze umfasst.

10. Die Nasen-Rachenkanüle gemäß Anspruch 7, **dadurch gekennzeichnet, dass** sie eine dritte Leitung zur Aufnahme einer Lichtquelle, wie zum Beispiel einer LED mit entsprechendem Stromversorgungskreis, mindestens einer Glasfaser zur Übertragung eines Lichtstrahls, der von einer externen Quelle erzeugt wird, und dergleichen umfasst.

11. Ein Nasen-Rachen-Beatmungskit, das Folgendes umfasst:
- einen schlauchförmigen Hauptkörper (2) aus weichem und verformbarem Material, versehen mit einer nichttraumatischen, d. h. weichen und gerundeten, distalen Spitze;
- einen Sensor (5), voreingestellt, um den Prozentsatz an Kohlendioxid zu messen, der im ausgeatmeten Gas vorhanden ist;
- eine Klammer (3), versehen mit mindestens einem Träger (4) für den Sensor (5); und
- einen echten kapnometrischen Wandler;
- wobei im Gebrauch die Klammer (3) verschiebbar und drehbar mit den Seitenwänden des schlauchförmigen Körpers (2) gekoppelt ist, zum Zwecke ihrer Anordnung in einer beliebigen geometrischen Konfiguration im Verhältnis zu dem schlauchförmigen Körper (2);
- wobei der Träger (4) von der Klammer (3) in einer im Wesentlichen radialen Richtung im Verhältnis zu dem schlauchförmigen Körper (2) absteht, wobei der Träger (4) einen Sitz (11) für die Aufnahme des Sensors (5) umfasst; wobei in der Nutzungskonfiguration, wenn der schlauchförmige Körper (2) in ein Nasenloch (A) eines Patienten (P) eingeführt wird, bis ein vorderes Ende (10) mit dem Rachen des Patienten (P) fluchtet, der Sensor (5) zumindest teilweise in das andere Nasenloch (B) des Patienten (P) eingeführt und von dem ausgeatmeten Luftstrom getroffen wird; wobei der Sensor (5) aus einer Kanüle besteht, durch welche das vom Patienten (P) ausgeatmete Gas entnommen werden und an den echten kapnometrischen Wandler geleitet werden kann.

## Revendications

1. Canule nasopharyngée comportant un corps tubulaire principal (2) constitué d'un matériau souple et déformable, un capteur (5) et une attache (3), ladite attache (3) étant pourvue d'au moins un support (4) pour ledit capteur (5), dans la configuration d'utilisation dudit corps tubulaire (2) étant insérée à l'intérieur d'une narine (A) d'un patient (P) jusqu'à ce qu'un avant (10) soit aligné avec le pharynx dudit patient (P), et ledit capteur (5), préréglé pour mesurer le pourcentage de dioxyde de carbone qui est présent dans le gaz exhalé, étant inséré au moins partiellement dans l'autre narine (B) du patient (P) et étant frappé par le flux exhalé, ladite attache (3) étant couplée de manière coulissante et rotative aux parois latérales dudit corps annulaire (2) pour son agencement dans toute configuration géométrique par rapport audit corps tubulaire (2), ledit support (4) faisant saillie à partir de ladite attache (3) dans une direction sensiblement radiale par rapport audit corps tubulaire (2), ledit support (4) comportant un logement (11) pour la réception dudit capteur (5), ledit capteur (5) étant constitué d'une canule à travers laquelle il est possible d'aspirer le gaz exhalé par le patient (P) et de l'envoyer vers un transducteur capnométrique réel.

2. Canule nasopharyngée selon la revendication 1, **caractérisée en ce que** ledit corps tubulaire (2) est pourvu d'au moins un conduit (6) qui a l'extrémité initiale (7) pouvant être associée, au moyen d'au moins un tube de raccordement (8) respectif, à des unités opérationnelles externes et à l'extrémité terminale (9) constituée d'une ouverture sur l'avant (10) du bord terminal dudit corps tubulaire (2).

3. Canule nasopharyngée selon la revendication 1, **caractérisée en ce que** ledit logement (11) a une forme de fente pour la réception stable dudit capteur (5) en fonction d'une pluralité de différentes configurations de différente distance dudit capteur (5) par rapport à la surface externe dudit corps tubulaire (2).

4. Canule nasopharyngée selon la revendication 1, **caractérisée en ce que** ladite attache (3) est sensiblement en forme de C et est constituée d'un matériau élastiquement déformable, pour un couplage détachable, en conséquence d'une déformation élastique de celle-ci, audit corps tubulaire (2).

5. Canule nasopharyngée selon la revendication 1, **caractérisée en ce que** ladite attache (3) est constituée d'une plaque de toute forme pourvue d'un trou central avec une forme et des dimensions qui sont complémentaires de celles dudit corps tubulaire (2) pour la réception coulissante dudit corps tubulaire (2).

6. Canule nasopharyngée selon la revendication 2, **caractérisée en ce que** ledit au moins un tube de raccordement (8) du au moins un conduit (6) comporte une borne pour un couplage à une unité respective pour distribuer les substances telles que des médicaments, des anesthésiques, des analgésiques, de l'eau et des mélanges de ceux-ci.

7. Canule nasopharyngée selon la revendication 2, **caractérisée en ce que** l'ouverture terminale (9) du au moins un conduit (6) comporte une buse pour atomiser les substances délivrées par celle-ci.

8. Canule nasopharyngée selon la revendication 7, **caractérisée en ce que** ladite buse d'atomisation est un corps creux qui est inséré à l'intérieur du au moins un conduit (6) et est agencée sensiblement sur l'ouverture terminale (9) respective, la cavité interne dudit corps creux a une forme qui converge initialement **et** diverge ensuite, tel qu'un tube à venturi, pour l'accélération des substances qui s'écoulent à l'intérieur d'elle avec une meilleure atomisation conséquente de celles-ci.

9. Canule nasopharyngée selon la revendication 1, **caractérisée en ce que** ledit corps principal (2) est constitué de polymères thermoplastiques et comporte un embout distal non traumatique, c'est-à-dire souple et arrondi.

10. Canule nasopharyngée selon la revendication 7, **caractérisée en ce qu'**elle comporte un troisième conduit pour la réception d'une source de lumière, telle qu'une LED avec un circuit d'alimentation respectif, au moins une fibre optique pour acheminer un faisceau de lumière produit par une source externe, et analogue.

11. Kit de ventilation nasopharyngée, comportant
- un corps tubulaire principal (2) constitué d'un matériau souple et déformable, pourvu d'un embout distal non traumatique, c'est-à-dire souple et arrondi,
- un capteur (5) préréglé pour mesurer le pourcentage de dioxyde de carbone qui est présent dans le gaz exhalé,
- une attache (3), pourvue d'au moins un support (4) pour un dit capteur (5), et
- un transducteur capnométrique réel,
- en utilisation, ladite attache (3) étant couplée de manière coulissante et rotative aux parois latérales dudit corps tubulaire (2) pour son agencement dans toute configuration géométrique par rapport audit corps tubulaire (2),
- ledit support (4) faisant saillie à partir de ladite attache (3) dans une direction sensiblement radiale par rapport audit corps tubulaire (2), ledit support (4) comportant un logement (11) pour la réception dudit capteur (5),
dans la configuration d'utilisation avec ledit corps tubulaire (2) inséré dans une narine (A) d'un patient (P) jusqu'à ce qu'un avant (10) soit aligné avec le pharynx dudit patient (P), ledit capteur (5) est inséré au moins partiellement dans l'autre narine (B) du patient (P) et étant frappé par le flux exhalé, ledit capteur (5) étant constitué d'une canule à travers laquelle il est possible d'aspirer le gaz exhalé par le patient (P) et de l'envoyer vers ledit transducteur capnométrique réel.
